**Europäisches Patentamt**

**European Patent Office**

**Office européen des brevets**

(19)

(11) Publication number:

**0 070 276**
**B1**

(12)

# EUROPEAN PATENT SPECIFICATION

(45) Date of publication of patent specification: **02.07.86**

(51) Int. Cl.⁴: **A 61 B 17/10, A 61 B 17/12**

(21) Application number: **81901822.7**

(22) Date of filing: **26.01.81**

(86) International application number:
**PCT/US81/00125**

(87) International publication number:
**WO 82/02487 05.08.82 Gazette 82/19**

(54) **SURGICAL DEVICE.**

(43) Date of publication of application:
**26.01.83 Bulletin 83/04**

(45) Publication of the grant of the patent:
**02.07.86 Bulletin 86/27**

(84) Designated Contracting States:
**CH FR LI**

(56) References cited:
**FR-A- 325 704**
**US-A-2 194 748**
**US-A-3 047 874**
**US-A-3 230 758**
**US-A-3 646 801**
**US-A-3 844 289**
**US-A-4 166 466**

(73) Proprietor: **Blake, Joseph Walter III**
**88 Main Street**
**New Canaan, CT 06840 (US)**

(73) Proprietor: **Kaufman, Jack William**
**357 Frankel Boulevard**
**Merrick, NY 11566 (US)**

(72) Inventor: **Blake, Joseph Walter III**
**88 Main Street**
**New Canaan, CT 06840 (US)**
Inventor: **Kaufman, Jack William**
**357 Frankel Boulevard**
**Merrick, NY 11566 (US)**

(74) Representative: **Madgwick, Paul Roland et al**
**Ladas & Parry Isartorplatz 5**
**D-8000 München 2 (DE)**

# Description

## Background of the Invention
### Field of the Invention

This invention relates to surgical devices in general.

More particularly, the invention relates to a magazine-loaded suturing device of the type using suturing clips, and to a magazine for use with such a device.

Still more specifically, this invention relates to a suturing device and a magazine of the type under discussion, wherein the suturing clips are automatically advanced to their position of use.

### The Prior Art

Certain types of suturing are still, and probably always will be, performed by hand. Other types of suturing, however, such as skin sutures and hemostatic sutures, are performed with the aid of stapel-like metal clips which are driven into the skin or, in the case of hemostatic clips, tightly squeezed about a blood vessel to stop the flow of blood through the same.

The invention will hereafter be described for purposes of explanation with reference to hemostatic applications. It should be understood, however, that it has more general applicability in the field of surgical suturing. It should also be understood that the term "suturing" as used herein is intended to carry the broadest meaning, i.e. to embrace hemostatic, skin, as well as other types of suturing in which suturing clips are employed.

In the prior art it is known to employ e.g., hemostatic forceps which are individually loaded with clips. In practice this means that at least two forceps may be used during the suturing operation: while the surgeon applies a hemostatic clip to a blood vessel with one forceps, an assistant (nurse) standing behind him inserts a single clip between the jaws of the second forceps, ready for use. The surgeon hands the used forceps back over his shoulder to be reloaded and the assistant hands him the newly loaded forceps in exchange. This exchange continues until suturing is completed. It is self-evident that this procedure is not very efficient, it ties up the assistant who could be performing some other function, and (particularly important in an emergency) is slows the surgeon down.

A proposal has been made to provide a magazine-loaded hemostatic forceps with semiautomatic clip feed. This has not found acceptance because the surgeon is required to perform a "cocking" operation after each clip is set; it is this operation which advances the next clip to operative position. Aside from the obvious disadvantage inherent in the lack of a fully automatic feed, the cocking motion is cumbersome and puts a strain on the surgeon's hands, which is definitely undesirable in terms of his steadiness and accuracy.

Solutions proposed in the stapler art, for devices used in closing boxes and the like, have been found not to be workable in the medical field. Suturing equipment is precison surgical equipment which must be small (there is the problem of access to certain wound areas), light (so as not to tire the surgeon and to permit ready manipulation) and sterilizable. The known staple solutions cannot be successfully integrated in equipment having to meet these requirements. A drastic departure from the prior art solutions is therefore required to arrive at the desired goal.

U.S. Patent 2,194,748 is concerned with a clip applicator in which a bank or nested string of clips remain stationary and a handle controlled carriage and feed bar mechanism detaches the leading clip while all remaining clips in the string continue to remain stationary.

One aspect of the invention resides in a surgical device, particularly a hemostatic device.

According to this aspect of the invention there is provided a surgical device for applying clips, said device including an applicator having a pair of cooperating jaws movable between an open and a closed position, anvils carried by said jaws and arranged to receive and cinch a clip, and a supply cartridge for feeding surgical clips to said jaws, said cartridge including a housing adapted to accommodate a string of clips and means for advancing the string towards the jaws for seriatim insertion of the respectively leading clip of the string between said anvils while the jaws are in said open position so as to become cinched between said anvils when the jaws move to said closed position, characterized in that a retractor is provided for retracting all except the respectively leading clip in the direction away from said anvils prior to movement of said anvils from said open towards said closed position.

The applicator may be made as metal stampings to reduce manufacturing cost since primarily only the jaws will require precision tolerances. However, it is also conceivable to use synthetic plastic materials, or to make the applicator in part of metal (e.g. the jaws) and in part of synthetic plastic material.

According to a further aspect of the invention there is provided a cartridge for use with a surgical device, particularly for use with hemostatic forceps, including a housing having a track provided with an outlet, and means for advancing a string of surgical clips in direction towards said outlet so that the respectively leading clips of the string are sequentially discharged from the outlet to become cinched by jaws of the surgical device, characterized in that a retractor is included in the housing for retracting the clips located upstream of the respective leading clip counter to said direction and away from said outlet prior to cinching of the discharged leading clip so as to avoid interference by the upstream clips with such cinching.

The jaws are curved to one side of the general plane of the applicator so as to provide "presentation", i.e. to enable the surgeon to more readily observe his suturing operation. The cartridge is elongated and located in a plane parallel, or substantially parallel, to the general plane of the forceps and in which each of the clips is located in its entirety.

The novel features which are considered as characteristic for the invention are set forth in the appended claims. Both the construction and method of operation of the invention as well as additional objects and advantages thereof, will however, be best understood from the following description of specific embodiments in connection with the accompanying drawings.

Brief Description of the Drawing

Fig. 1 is a plan view showing one side of a forceps;

Fig. 2 is a side view showing only the jaw area of the forceps in Fig. 1;

Fig. 3 is a fragmentary close-up view of the jaws in Fig. 2, partly section;

Fig. 4 is a plan view of a surgical clip to be used with the forceps of Figs. 1—3;

Fig. 5 is a somewhat diagrammatic side view of a cartridge according to the invention, shown in assembled condition;

Fig. 6 is a plan view of the underside, showing the cartridge elements in disassembled condition;

Fig. 7 is a section on line VII—VII of Fig. 8;

Fig. 8 is a diagrammatic perspective view of the assembled cartridge;

Fig. 9 is a fragmentary perspective view with parts omitted for clarity;

Fig. 10 is a fragmentary perspective view, showing a detail of the front end of the cartridge; and

Figs. 11A—11C are simplified action diagrams showing the operation of the cartridge.

Description of Preferred Embodiments

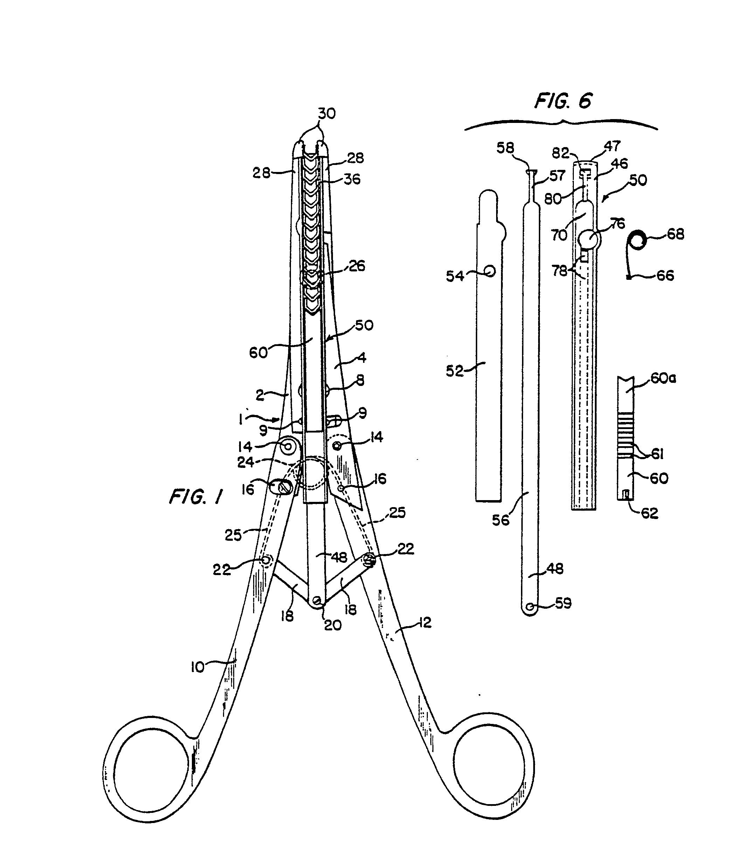

An applicator for applying the cartridge-supplied clips is illustrated in Figs. 1—3 in form of a hemostat forceps, by way of explanation. Reference numeral 1 identifies the forceps in toto. In the usual manner the forceps 1 has two arms 2, 4 which are pivotally connected by means of a pivot (not shown) on one and a transverse slot 8 in the other arm. The arm 4 is also provided with a pair of transversely spaced cartridge retaining and stabilizing stops 9 and with a hole 26 which receives a pivot (to be discussed infra) of the cartridge.

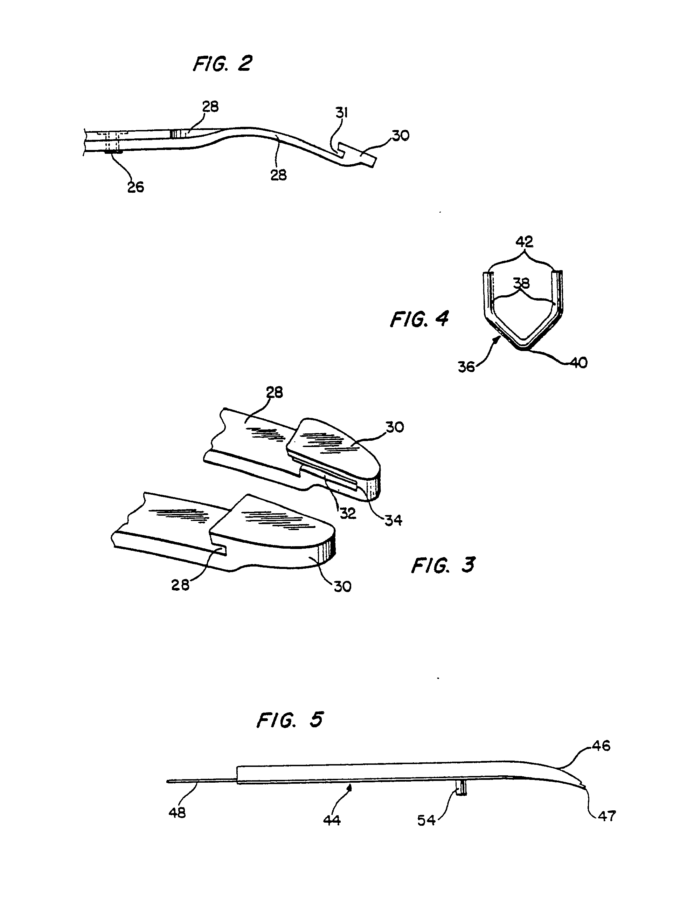

Arms 2, 4 have respective handles 10, 12 which, in this particular embodiment, are separate elements pivoted to the arms 2, 4 at pivots 14, 14. In addition, each handle 10, 12 is connected to the associated arm via a slot-and-pin connection 16 (one shown in detail in Fig. 1; the other is located at the other side of the forceps). The connections including the pivot, the slot 8 and the slot-and-pin connection 16 provide for a lost-motion movement when the arms 2, 4 are pivoted relative to one another in a sense causing the end section or anvil 30 of their curved (see Fig. 2) jaws 28 to approach one another. The significance of this lost-motion movement will become apparent as the description proceeds.

Handles 10, 12 are connected by a link 18 having its two bars pivoted to each other at 20 and to opposite sides of arms 10, 12, at 22, 22. A torsion spring 24 has two legs 25 connected to the pivots 22, 22 and thus permanently tends to urge the arms 2, 4 apart to the position illustrated in Fig. 1. Arms 10 and 12 are also urged to the open position.

Fig. 2 shows that the jaws 28 and their end sections 30 are curved to one side of the general plane of the forceps 1, in order to provide "presentation", i.e. to assure that the view of the wound area being worked upon by the surgeon is not obscured by any other part of the forceps. In Fig. 3 the end sections 30 will be seen to be provided in their facing surfaces with longitudinal grooves 32 for the surgical clips. These grooves are closed at their front ends, to provide end stops (abutments) 34 for the clips to be supplied by the magazine.

A single clip 36 is illustrated in Fig. 4 and will be seen to be of generally U-shaped outline in plan view, having two arms 38 which are connected by a bight 40 and have free ends 42 adapted to abut against the end stop 34.

It should be understood that the illustration in Fig. 4 is by way of example only, and that a clip suitable for use in the cartridge of the invention may depart in various ways from the shape shown in Fig. 4 and still be suitable.

Details of an exemplary cartridge, for use with the applicator of Figs. 1—3, will now be described with reference to Figs. 4—11.

Fig. 5 is a somewhat diagrammatic side view, showing the cartridge 44 in assembled condition and illustrating its curved front end portion 46 (the curved is complementary to that of the forceps jaw 28) and a portion 48 of a clip retractor which extends out from the back end of the cartridge. A pivot 54 is to be inserted into the hole 26 of the forceps 1.

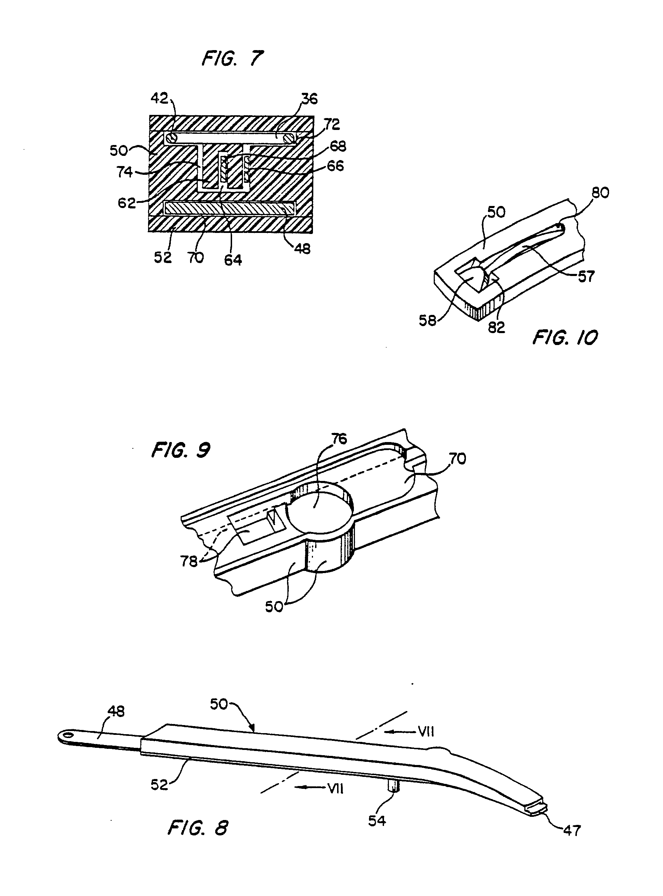

The individual elements of the cartridge 44 are shown in Fig. 6, which illustrates the cartridge in a plan view and in disassembled form. The main cartridge body is identified with reference numeral 50, and in the assembled condition, will be covered by the illustrated backing plate 52 on which the previously mentioned pivot 54 is provided (of one piece with it or suitably secured to it). The clip retractor is identified with reference numeral 56; it is a length of e.g. spring steel whose purpose will become clear in due course. The front portion 57 of the retractor 56 is of reduced width and carries at its free end a head 58 of e.g. generally triangular shape. The rear portion 48 (see Fig. 5) is provided with means 59 (e.g. a hole, a projection or the like) for connecting it to the center pivot 20 of link 18.

The cartridge body 50 will accommodate a string of clips 36 is unconnected form; i.e. they will be sequentially but individually located in a clip track of the body 50, to be fed forwardly (to the front end 46) by spring action. For this purpose a clip follower 60 is provided, in form of a strip-shaped element which is provided, in its front end with a notch shaped to accommodate the bight of the last clip 36 in the string. The rear (trailing) end of follower 60 has a tail piece 62 projecting to one major side of the follower (compare Fig. 7) and provided with a slot 64 in which a hook-shaped end portion 66 of the clip-biasing spring 68 is engageable. At least the center portion of follower 60 is provided with transversely extending ridges and grooves 61, so as to make the follower more flexible and enable it to follow the curvature of the front end 46 of the cartridge.

The spring 68 is a constant-force coil spring (commercially available under the tradename "Neg-A-Tor") which exerts constant forward spring force upon the follower 60 (and via the same upon the string of clips 36) regardless of the degree of extension of the spring. In other words: the tension exerted upon the clips 36 when a full string is present in the cartridge and the spring is extended to its maximum, is the same as the tension exerted upon the last remaining clip in the cartridge after all other clips in the string have already been depressed and used.

That side of body 50 which will face towards the forceps 1 when the cartridge 44 is installed on the same, is formed with a recessed track 70 in which the clip retractor 56 slides; in the assembled condition of the cartridge this track 70 is covered by plate 52. Toward the front end of the cartridge the track 70 merges into a slot 80 slightly wider than the portion 57 of the retractor; this slot 80 extends through the cartridge body 50 and opens into a clip track 72 (Fig. 7) which is formed in the other side of body 50, i.e. the side which faces away from the forceps 1.

A cavity 76 is formed in that side of body 50 which has the retractor track 70 and has an open end flush with the bottom surface of track 70 (Fig. 9); i.e. the cavity 76 is recessed below the level of track 70. Also formed in the body 50, offset to one side (Fig. 7) are a track and an opening 78 in which the extended portion of spring 68 and the tail 62 of the follower 60 move. The follower itself moves in the clip track 72 behind the string of clips and its tail extends into the track 78 which is open to the clip track 72.

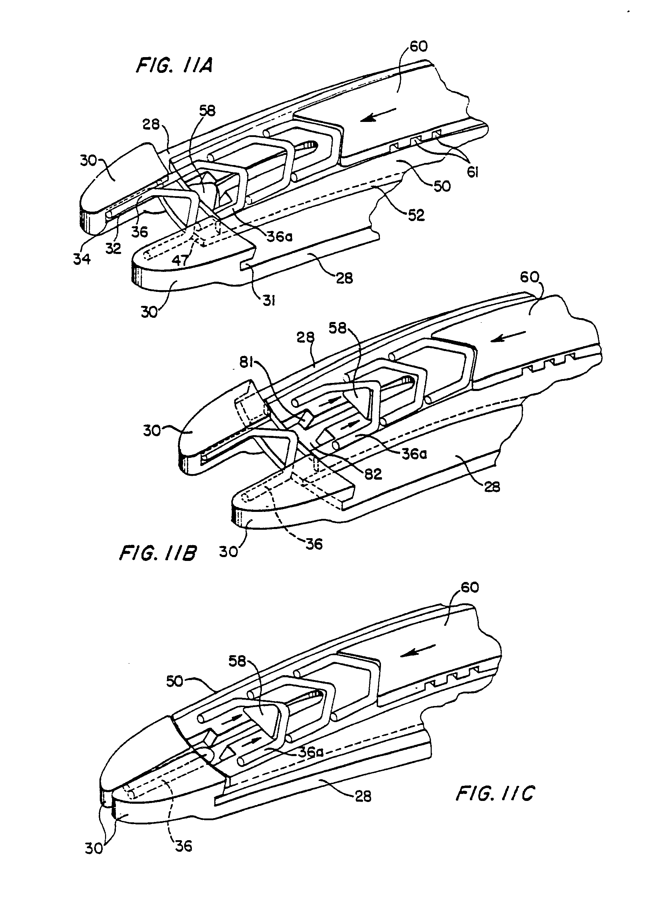

Near its front end the body 50 is provided, at the front of slot 80 and in the bottom surface of the clip track 72, with a recess 82 dimensioned to accommodate the head 58 of retractor 56, so that the head, when located in the recess 82, will be flush with (or recessed below) the aforementioned bottom surface and not interfere with the forward movement of the clips 36 which move right over and past it. Inclined ramps or camming surfaces 81 (Fig. 11B) are located at either side of slot 80 and rise out of the recess to the level of the bottom surface of the clip track 72.

The portion 46 of the cartridge 44, or rather of the body 50 in particular, is curved as illustrated in Fig. 5. This curvature follows that of the forceps jaws 28 and its purpose is to allow the portion 46 to snugly fit against the jaws 28. Correspondingly, the retractor track 70 and the clip track 72 are also curved. The front portion 57 of the retractor 56, however, is curved more strongly than the front portion of the tracks 70, 72. The portion 57 of retractor 56 extends through the slot 80 from the track 70 into the track 72 (i.e. from one side of the body 50 to the other as shown in Fig. 10). The spring-back tendency of the portion 57 (the retractor is of spring steel or the like) is counter to this deflection from track 70 into track 72. Consequently, the head 58 is always biased into contact with the bottom surface of track 72. Therefore the head 58 snaps into the recess 82 when the retractor is far enough forward to permit this, i.e. when head 58 is in registry with the recess. In this position the head is located beneath the path of the clips advancing in track 72, so as not to interfere with them. When the retractor is moved backwards, however, the head 58 slides up on the ramps 81 and enters into track 72; i.e. it becomes located in the path of the clips 36. More specifically, the head then enters into the space between the legs 38 of the upstream clip 36a which is located immediately behind the leading clip (Fig. 11A), and upon further retraction of retractor 56 the head 58 engages the inside of the bight 40 of this upstream clip 36a and retracts the same (and thereby all other clips behind it) away from the front end of the cartridge. The purpose is to move clip 36a out from between the closing jaws 28, 30 (Fig. 11B) so that these can cinch the leading clip 36 between them (e.g. about a blood vessel to occlude the same) as shown in Fig. 11C. When the jaws re-open the retractor 56 slides forwardly, head 58 snaps back into recess 82 and the next clip (i.e. 36a) can now enter between the portions (anvils) 30 of jaws 28.

The operation of the device will be understood from what has been discused thus far:

The cartridge 44 is mounted to the forceps 1 by placing its portion 47 into the notches 31 of anvils 30, inserting pivot 54 into hole 26 and connecting means 59 to the center pivot 20 of link 18. When the handles 10, 12 are now squeezed towards one another the two parts of link 18 pivot about the center pivot 20 and the end pivots 22; since center pivot 20 is located rearwardly of a line connecting the end pivots 22, the approach of the end pivots 22 towards one another has the effect of shifting the center pivot 20 still farther rearwards of this line. The retractor 56 is connected to the center pivot and such rearward shifting therefore causes it to slide backwardly, so that head 58 moves from the positon of Fig. 11A to that of Fig. 11C. Since the cartridge has just been newly installed, no clip will at this time be located between anvils 30 so that only a retraction of the string of clips takes place in the cartridge.

As soon as the pressure on the handles 10, 12 is released, they are forced apart (to the Fig. 1 position) by the spring 24. In so doing the pivot 20 moves forwardly and allows the spring 68 to assist in sliding retractor 56 to forward position (by exerting pressure on head 58 via the string of clips bearing upon it). When head 58 reaches recess 82 it snaps into the same, thereby releasing the leading clip 36 which it has previously held, for advancement into the grooves 32 of anvils 30, until the free ends 42 of its legs 38 come to rest against the end stops 34. During this movement the next-following clip (e.g. clip 36a) has advanced with parts of its own legs between the anvils 30 and would prevent proper operation of the device, if allowed to remain in place. In order, however, to affix the leading clip 36 (e.g. about a blood vessel), the user must again squeeze handles 10, 12 together so as to crush (crimp) the clip between the anvils 30. In doing so he automatically effects a retracting of all clips (36a, etc.) located behind leading clip 36 (Fig. 11B), so that the device can operate as intended (Fig. 11C). Each successive squeezing of the handles 10, 12 will apply another clip to the wound with the next-following clip moving into readiness between the anvils 30 as pressure on the handles is relaxed between squeezes. This continues until cartridge 44 is empty.

If, after the last clip is used, the now empty anvils 30 were allowed to press directly upon e.g. a blood vessel, they would do severe damage to the same. The invention avoids the possibility by making the follower 60 so long that its front end portion 60a enters into the grooves 32 of anvil 30 after the last clip is used. In other words: as the spring 68 returns to its rest position after the last clip is applied, it urges the front end portion 60a into the grooves 32. This prevents the anvils 30 from being closed directly (i.e. without interposition of a clip) about the blood vessel and, at the same time, tells the surgeon (who can no longer squeeze the handles together) that the cartridge is empty.

The cartridge 44 is held to the forceps 1 at undercuts 31 and at the pivot 20. It therefore can shift relative to the forceps to a limited extent, in a plane parallel to the general plane of the forceps. The degree of such movement is limited by the abutments 9 between which the cartridge is located. The front end 47 can self-adjust relative to the anvils 30 during use of the forceps, as a comparison of Figs. 11A—11C indicates.

The purpose of the lost motion afforded by the manner in which handles 10, 12 are mounted on arms 2, 4 is to cause (during initial squeezing of the handles 10, 12) the retraction of the retractor 56 and thus of the clips 36, before the jaws 28 and their anvils 30 begin to move toward one another. This allows clip 36a to move out of the way of anvils 30.

It will be appreciated that in the region of the spring cavity 76 the clip track 72 will be separated from the cavity 76 by a thin wall which avoids interference between the clips and the spring.

It will also be appreciated that the disclosed embodiment is susceptible to various modifications without departing from the concept of the invention. For example, the handles 10, 12 could be made of one piece with (or at least rigidly connected to) the arms 2, 4. The lost motion would then be provided by using (e.g. at the location of hole 26) a transverse slot-and-pin connection for the arms 2, 4. The pin itself could be hollow and its hollow interior serve as a replacement for the hole 26. A retainer could be provided for preventing the clip located in the grooves of the anvils from sliding back into the cartridge when the upstream clips are retracted and in the event the surgeon then holds the forceps with the jaws in elevated position.

The spring 24 and links 18 could be replaced by a one-piece unit, e.g. a generally chevron-shaped spring having its ends rigidly connected to the arms 10, 12 and yielding elastically in essentially the same sense as the links 18, so that its center (corresponding to pivot 20) would recede from and approach the jaws 28 in essentially the same manner as the pivot does. The cartridge may also be provided with a releasable retainer for holding the first (lead) clip against being expelled from the cartridge under the pressure of spring 68 until the cartridge is installed in the forceps.

The cartridge and/or forceps may be made wholly or in part of synthetic plastic material. Suitable plastic materials, especially for the cartridge, are polysulfone which is a high-strength autoclavable plastic approved by the Food and Drug Administration for medical use; polycarbonates (e.g. "Lexan"/TM) or acetal (e.g. "Delrin"/TM) which have lesser qualities but are also considerably less costly than the preceding materials. Such plastics may, of course, be reinforced by e.g. carbon fibers or the like.

The cartridge will be disposable, and the forceps may be permanent. The parts of the latter may be stamped from sheet metal (e.g. brass, stainless steel) to reduce manufacturing costs. However, the forceps could itself be disposable together with the cartridge, if it can be made inexpensively enough. For example, the main parts of the forceps except for the arms 2, 4 might be made of synthetic plastic, or even the arms themselves might be made thereof and perhaps only the anvils made of metal. This is primarily a question of using a plastic having sufficient strength to withstand the forces acting upon it during the application (crimping or cinching) of the clips. Arm 2 and 4 may be made of synthetic plastic material and have metal reinforcements.

As mentioned before, the invention — i.e. the cartridge and/or the forceps — is of particular advantage for use in ligating blood vessels. However, the invention is also suitable for use in general suturing and surgical stapling. The shape (outline) of the clips may be varied as required and the free ends of the clips may be pointed for special non-ligating applications. The connection of the retractor 48 to the pivot 20 may be by way of a hole in the retractor (or the link and a pin on the link of the retractor). However, it goes without saying that other connections may be used, such as a snap-type coupling, a wedging coupling, or the like.

What is claimed as new and desired to be protected by Letters Patent is set forth in the appended claims.

## Claims

1. A surgical device for applying clips, said device including an applicator having a pair of cooperating jaws (28) movable between an open and closed position, anvils (30) carried by said jaws (28) and arranged to receive and cinch a clip (36), and a supply cartridge (44) for feeding surgical clips (36, 36a) to said jaws, said cartridge including a housing (50) adapted to accommodate a string of clips and means (60) for advancing the string towards the jaws for seriatim insertion of the respectively leading clip (36) of the string between said anvils (30) while the jaws (28) are in said open position so as to become cinched between said anvils when the jaws move to said closed position, characterized in that a retractor (56) is provided for retracting all except the respectively leading clip (36) in the direction away from said anvils (28) prior to movement of said anvils from said open towards said closed position.

2. A surgical device according to claim 1, characterized in that the retractor (56) and advancing means (60) are accommodated in the cartridge housing (50), said housing having a leading end section and a trailing end section and first and second substantially parallel tracks (72, 70) both extending between said end sections said advancing means incorporating a spring biased follower (60) for biasing a string of clips in said first track (72) lengthwise toward said leading end section, and said retractor (56) being accommodated in said second track (70) and having a leading portion extending into and movable in said first track (72) between one position in which said leading portion is disengaged from said clips and permits the respectively leading clip to issue from said leading end section and another position in which said leading portion engages the clip immediately upstream of the leading clip and retracts it, and thereby said string of clips, in the direction towards said trailing end section.

3. A surgical device according to claim 2, characterized in that a constant-force coil spring (68) engages said spring biased follower (60) to urge said follower in the first track (72) in a direction toward said leading end into contact with a trailing clip of said string, and in that said retractor (56) is an elongated strip-member extending through said housing (50) slidable in said second track (70) and has a trailing end portion located adjacent said trailing end, at least said leading portion being of spring material and having a permanent bias tending to deflect it from said second track towards said first track.

4. A surgical device according to claim 3, characterized in that the applicator has a general plane and the jaws are curved out of said general plane to one side thereof, said leading end section of said cartridge housing (50) being curved in substantial correspondence with said jaws, and in that the cartridge (44) is detachably mounted on the applicator for pivotal movement about a pivot axis extending substantially normal to said general plane by a pivot pin (54) on said housing (50) and a socket (26) on said applicator and dimensioned to receive said pivot pin.

5. A surgical device according to any of claims 2 to 4, characterized in that the housing (50) has first and second substantially parallel major surfaces which respectively have said tracks recessed therein, a slot (80) extending lengthwise of said tracks in said leading end section and extending between said tracks, and a recess (82) at a forward end of said slot below the level of said first track and connected therewith by a camming surface (81) which is inclined upwardly towards said level and rearwardly toward the trailing end section and in that said retractor (56) is strip-shaped and has at least a front portion which is of spring material and bent to extend through the slot (80) from said second track into said first track said front portion having a head (58) which enters into said recess (82) and becomes located below the level of said first track when said retractor slides towards said leading end section and which rides up on said camming surface (81) to enter said first track when said retractor slides towards said trailing end section.

6. A surgical device according to claim 5, characterized in that the applicator includes a pair of handles (10, 12) which approach one another when said jaws move toward said closed position and recede from one another when said jaws move toward said open position, and in that the retractor (56) is detachably linked to said handles for sliding movement towards said leading end section as a function of the handles approaching one another, and vice versa.

7. A surgical device according to any of claims 1 to 6, characterized in that said jaws are blocked against movement to said closed position when no clips remain in the cartridge housing.

8. A cartridge (44) for use with a surgical device, according to one of the claims 1 to 7 particularly for use with hemostatic forceps, including a housing (50) having a track (72) provided with an outlet, and means for advancing a string of surgical clips (36, 36a) in direction towards said outlet so that the respectively leading clips of the string are sequentially discharged from the outlet to become cinched by jaws of the surgical device, characterized in that a retractor (56) is included in the housing (50) for retracting the clips located upstream of the respective leading clip counter to said direction and away from said outlet prior to cinching of the discharged leading clip so as to avoid interference by the upstream clips with such cinching.

9. A cartridge according to claim 8, characterized in that the retractor (56) and advancing means (60) are accommodated in the housing (50), said housing having a leading end section and a trailing end section and first and second substantially parallel tracks (72, 70) both extending between said end sections, said advancing means incorporating a spring biased follower (60) for biasing a string of clips in said first track (72) lengthwise toward said leading end section, and said retractor (56) being accommodated in said second track (70) and having a leading portion extending into and movable in said first track (72) between one position in which said leading portion is disengaged from said clips and permits the respectively leading clip to issue from said leading end section and another position in which said leading portion engages the clip immediately upstream of the leading clip and retracts it, and thereby said string of clips, in the direction towards said trailing end section.

10. A cartridge according to claim 9, characterized in that the advancing means includes a follower (60) slidable in said first track (72) and a constant-force coil spring (68) engaging said follower (60) to urge said follower in the direction toward said leading end into contact with a trailing clip of said string.

**Patentansprüche**

1. Chirurgisches Instrument mit einer Einsetzvorrichtung, die ein Paar von miteinander zusammenwirkenden Backen (28) besitzt, die zwischen einer Offen- und einer Schließstellung bewegbar sind, ferner von den Backen (28) getragene Ambosse (30) zur Aufnahme und zum Umbiegen einer Klemme (36), und mit einer Vorratspatrone (44) zum Vorschieben von chirurgischen Klemmen (36, 36a) zu den Backen, wobei die Patrone ein Gehäuse (50) besitzt, das zur Aufnahme einer Reihe von Klemmen geeignet sit, und eine Einrichtung (60) zum Vorschieben des Bandes zu den Backen hin derart, daß die jeweils vorderste Klemme (36) des Bandes zwischen die Ambosse (30) eingeführt wird, während sich die Backen (28) in der offenen Stellung befinden, so daß bei der Bewegung der Backen in die Schließstellung die Klemme zwischen den Ambossen geschlossen wird, dadurch gekennzeichnet, daß eine Rückzieheinrichtung (56) vorgesehen ist, die dazu dient, vor der Bewegung der Ambosse aus der Offenstellung zu der Schließstellung hin alle Klemmen außer der jeweils vordersten Klemme (36) von den Ambossen (28) weg zurückzuziehen.

2. Chirurgisches Instrument nach Anspruch 1, dadurch gekennzeichnet, daß die Rückziehvorrichtung (56) und die Vorschubeinrichtung (60) in dem Patronengehäuse (50) angeordnet ist, das einen vorderen und einen hinteren Endteil besitzt und eine erste und eine zweite Führung (72, 70), die sich im wesentlichen parallel zueinander zwischen den genannten Endteilen erstrecken, wobei die Vorschubeinrichtung einen federbelasteten Stößel (60) besitzt, der eine in der ersten Führung (72) befindliche Reihe von Klemmen in der Längsrichtung zu dem vorderen Endteil hin belastet, und daß die Rückzieheinrichtung (56) in der zweiten Führung (70) angeordnet ist und einen vorderen Teil besitzt, der sich in die zweite Führung (72) erstreckt und in dieser zwischen einer Stellung, in der der genannte vordere Teil von den Klemmen abgerückt ist und einen Austritt der jeweils vordersten Klemme aus dem vorderen Endteil gestattet, und einer anderen Stellung bewegbar ist, in der vordere Teil an der der vordersten Klemme unmittelbar folgenden Klemme angreift und diese und damit die Reihe der Klemmen zu dem hinteren Endteil hin zurückzieht.

3. Chirurgisches Instrument nach Anspruch 2, dadurch gekennzeichnet, daß an dem Stößel in der ersten Führung (72) eine kraftkonstante Feder (68) angreift, die den Stößel zu dem vorderen Ende hin bis zur Anlage an der hintersten Klemme der Reihe zu bewegen trachtet, daß die Rückzieheinrichtung (56) ein Stab ist, der das Gehäuse (50) durchsetzt, in der zweiten Führung (70) verschiebbar ist und einen dem genannten hinteren Ende benachbarten, hinteren Endteil besitzt, und daß mindestens der vordere Teil aus einem Federwerkstoff besteht und dauernd unter einer Vorspannung steht, die trachtet, diesen vorderen Teil von der zweiten Führung zu der ersten Führung hin auszulenken.

4. Chirurgisches Instrument nach Anspruch 3, dadurch gekennzeichnet, daß die Einsetzvorrichtung eine Hauptebene besitzt, daß die Backen aus der Hauptebene zu einer Seite derselben gekrümmt sind, daß der vordere Endteil des Patronengehäuses (50) im wesentlichen wie die Backen gekrümmt ist, und daß die Patrone (44) auf der Einsetzvorrichtung abnehmbar montiert und um eine Schwenkachse verschwenkbar ist, die zu der Hauptebene im wesentlichen normal ist und von einem Schwenkzapfen (54) gebildet wird, der an dem Gehäuse (50) vorgesehen ist, und von einer an der Einsetzvorrichtung vorgesehenen Fassung (26), die so bemessen ist, daß sie den Zapfen aufnehmen kann.

5. Chirurgisches Instrument nach einen der Ansprüche 2 bis 4, dadurch gekennzeichnet, daß das Gehäuse (50) eine erste und eine zweite Fläche besitzt, die im wesentlichen parallel zueinander und mit je einer der genannten Führungen vertieft ausgebildet ist, daß sich in dem vorderen Endteil zwischen den Führungen ein Langloch (80) entlang den Führungen erstreckt, daß am vorderen Ende des Langloches eine Vertiefung (82) vorgesehen ist, die sich bis unter das Niveau der ersten Führung erstreckt und mit dieser durch eine Auslenkfläche (81) verbunden und zu dem genannten Niveau hin aufwärts und zu dem hinteren Endteil hin rückwärts geneigt ist, daß die Rückzieheinrichtung (56) stabförmig ist und mindestens in ihrem Vorderteil aus federndem werkstoff besteht und dieser Vorderteil so gebogen ist, daß er sich von der zweiten Führung durch das Langloch (80) in die erste Führung erstreckt, daß der Vorderteil einen Kopf (58) besitzt, der in die Vertiefung (82) eintritt und der unter dem Niveau der ersten Führung angeordnet ist, wenn die Rückzieheinrichtung zu dem vorderen Endteil hin gleitet, und daß der Kopf auf der Auslenkfläche (81) aufwärts in die erste Führung gleitet, wenn die Rückzieheinrichtung zu dem hinteren Endteil hin verschoben wird.

6. Chirurgisches Instrument nach Anspruch 5, dadurch gekennzeichnet, daß die Einsetzeinrichtung ein Paar von Griffen (10, 12) besitzt, die sich einander nähern, wenn wich die Backen zu der Schließstellung hin bewegen, und die sich voneinander entfernen, wenn sich die Backen zu der Offenstellung hin bewegen, und daß die Rückzieheinrichtung (56) mit den Griffen abnehmbar gelenkig verbunden ist, so daß sie zu dem vorderen Endteil hin gleitet, wenn die Griffe zueinander hin bewegt werden, und umgekehrt.

7. Chirurgisches Instrument nach einem der Ansprüche 1 bis 6, dadurch gekennzeichnet, daß die Backen gegen eine Bewegung in die Schließstellung blockiert sind, wenn in dem Patronengehäuse keine Klammern mehr vorhanden sind.

8. Patrone (44) für die Verwendung mit einem chirurgischen Instrument, insbesondere für die Verwendung mit Gefäßklemmen, mit einem Gehäuse (50), das eine Führung (72) besitzt, die mit einem Austritt versehen ist, und mit einer Einrichtung zum Vorschieben einer Reihe von chirurgischen Klemmen (36, 36a) zu dem Austritt hin, so daß die jeweils vorderste Klemme der Reihe aus dem Austritt ausgebracht und von Backen des chirurgischen Instruments geschlossen wird, dadurch gekennzeichnet, daß in dem Gehäuse (50) eine Rückzieheinrichtung (56) vorgesehen ist, die dazu dient, die der jeweils vordersten Klemme folgenden Klemmen in der von dem Austritt wegführenden Richtung züruckzuziehen, bevor die ausgebrachte vorderste Klemme geschlossen wird, so daß dieses Schließen nicht durch die folgenden Klemmen gestört wird.

9. Patrone nach Anspruch 8, dadurch gekennzeichnet, daß die Rückzieheinrichtung (56) und die Vorschubeinrichtung (60) in dem Patronengehäuse (50) angeordnet ist, das einen vorderen und einen hinteren Endteil besitzt und eine erste und eine zweite Führung (72, 70), die sich im wesentlichen parallel zueinander zwischen den genannten Endteilen erstrecken, wobei die Vorschubeinrichtung einen federbelasteten Stößel (60) besitzt, der eine in der ersten Führung (72) befindliche Reihe von Klemmen in der Längsrichtung zu dem vorderen Endteil hin belastet, und daß die Rückzieheinrichtung (56) in der zweiten Führung (70) angeordnet ist und einen vorderen Teil besitzt, der sich in die zweite Führung (72) erstreckt und in dieser zwischen einer Stellung, in der der genannte vordere Teil von den Klemmen abgerückt ist und einen Austritt der jeweils vordersten Klemme aus dem vorderen Endteil gestattet, und einer anderen Stellung bewegbar ist, in der vordere Teil an der der vordersten Klemme unmittelbar folgenden Klemme angreift und diese und damit die Reihe der Klemmen zu dem hinteren Endteil hin zurückzieht.

10. Patrone nach Anspruch 9, dadurch gekennzeichnet, daß die Rückzieheinrichtung einen in der ersten Führung (72) verschiebbaren Stößel (60) und eine kraftkonstante Schraubenfeder (68) besitzt, die an den Stößel (60) angreift und ihn bis zur Anlage an einer hinteren Klemme der Reihe zu dem vorderen Ende hin zu bewegen trachtet.

## Revendications

1. Dispositif chirurgical destiné à poser des agrafes, ledit dispositif comprenant un applicateur possédant une paire de mâchoires (28) coopérant entre elles qui peuvent de déplacer entre une position ouverte et une position fermée, des enclumes (30) portées par lesdites mâchoires (28) et agencées pour recevoir et serrer une agrafe (36), et une cartouche d'alimentation (44) servant à acheminer des agrafes chirurgicales (36, 36a) auxdites mâchoires, ladite cartouche comprenant un boîtier (50) adapté pour recevoir une file d'agrafes, et des moyens (60) servant à faire avancer la file vers les mâchoires pour insérer à chaque fois l'agrafe de tête (36) de la file entre lesdites enclumes (30) pendant que les mâchoires (28) sont en position ouverte, de manière qu'elle soit serrée entre lesdites mâchoires lorsque les mâchoires prennent ladite position fermée, caractérise en ce qu'un rétracteur (56) est prévu pour rétracter toutes les agrafes (36) sauf l'agrafe de tête, dans le sens qui s'éloigne desdites enclumes (28) avant le mouvement desdites enclumes allant de ladite position ouverte vers la position . fermée.

2. Dispositif chirurgical selon la revendication 1, caractérisé en ce que le rétracteur (56) et les moyens d'avance (60) sont logés dans le boîtier de cartouche (50), ledit boîtier possédant une section d'extrémité de tête et une section d'extrémité de queue, un premier et un deuxième couloirs (72, 70) sensiblement parallèles, qui s'étendent tous deux entre lesdites sections d'extrémités, lesdits moyens d'avance comprenant un curseur (60) sollicité par un ressort et destiné à solliciter une file d'agrafes logée dans ledit premier couloir (72), longitudinalement vers ladite section d'extrémité de tête, et ledit rétracteur (56) étant logé dans ledit deuxième couloir (70) et possédant une partie de tête qui pénètre dans ledit premier couloir (72) et peut se déplacer dans le premier couloir entre un position dans laquelle ladite partie de tête est dégagée desdites agrafes et permet à l'agrafe de tête respective de sortir de ladite section d'extrémité de tête, et une autre position, dans laquelle ladite partie de tête attaque l'agrafe située immédiatement en amont de l'agrafe de tête et la rétracte, et rétracte de cette façon ladite file d'agrafes, dans la direction allant vers ladite section d'extrémité de queue.

3. Dispositif chirurgical selon la revendication 2, caractérisé en ce qu'un ressort hélicoïdal à force constante (68) attaque ledit curseur (60) sollicité par ressort pour tendre à déplacer ledit curseur dans le premier couloir (72) dans une direction allant vers ladite extrémité de tête, pour le mettre en contact avec une agrafe de queue de ladite file, et en ce que ledit rétracteur (56) est un élément allongé en forme de bande, qui s'étend à travers ledit boîtier (50), qui peut coulisser dans ledit deuxième couloir (70), et qui possède une partie d'extrémité de queue située dans la région de ladite extrémité de queue, au moins ladite partie de tête étant faite d'une matière pour ressorts et possédant une sollicitation permanente qui tend à la faire passer dudit deuxième couloir vers le premier couloir.

4. Dispositif chirurgical selon la revendication 3, caractérisé en ce que l'applicateur possède un plan général et que les mâchoires sont incurvées endehors dudit plan général, vers un côté de ce plan, ladite section d'extrémité de tête dudit boîtier (50) de la cartouche étant incurvée selon une courbure correspondant sensiblement auxdites mâchoires, et en ce que la cartouche (44) est montée de façon détachable sur l'applicateur, pour décrire un mouvement de pivotement autour d'un axe pivot qui s'étend sensiblement normalement audit plan général, par un axe pivot (54) prévu sur ledit boîtier (50) et une cavité (26) prévue sur ledit applicateur et dimensionnée pour recevoir ledit axe pivot.

5. Dispositif chirurgical selon l'une quelconque des revendications 2 à 4, caractérisé en ce que le boîtier (50) possède une première et une deuxième grandes faces sensiblement parallèles dans lesquelles sont respectivement creusés lesdits couloirs, une fente (80) qui s'étend selon la longueur desdits couloirs, dans ladite section d'extrémité de tête et qui s'étend entre lesdits couloirs, et une cavité (82) située à l'extrémité avant de ladite fente, au-dessous du niveau dudit premier couloir et est reliée à celui-ci par une surface de came (81) qui est inclinée vers le haut vers ledit niveau et vers l'arrière, vers la section d'extrémité de queue, et en ce que ledit rétracteur (56) est en forme de bande et possède au moins une partie avant qui est faite d'une matière à ressorts et est recourbée pour s'étendre à travers la fente (80), en passant dudit deuxième couloir au premier couloir, ladite partie avant ayant une tête (58) qui pénètre dans ladite cavité (82) et est placée au-dessous du niveau dudit premier couloir lorsque ledit rétracteur coulisse vers ladite section d'extrémité de tête, et qui monte sur ladite surface de came (81) pour pénétrer dans ledit premier couloir lorsque ledit rétracteur coulisse vers ladite section d'extrémité de queue.

6. Dispositif chirurgical selon la revendication 5, caractérisé en ce que l'applicateur comprend une paire de poignées (10, 12) qui se rapprochent l'une de l'autre lorsque lesdites mâchoires se rapprochent de ladite position fermée et qui s'éloignent l'une de l'autre lorsque lesdites mâchoires se rapprochent de ladite position ouverte, et en ce que le rétracteur (56) est articulé de façon détachable sur lesdites poignées pour décrire un mouvement de coulissement vers ladite section d'extrémité de tête en fonction du rapprochement mutuel des poignées, et inversement.

7. Dispositif chirurgical selon l'une quelconque des revendications 1 à 6, caractérisé en ce que lesdites mâchoires sont bloquées contre le déplacement vers ladite position fermée lorsqu'il ne reste plus d'agrafes dans le boîtier de la cartouche.

8. Cartouche (44) destinée à être utilisée avec un dispositif chirurgical selon l'une des revendications 1 à 7, en particulier pour l'utilisation avec des pinces hémostatiques, comprenant un boîtier (50) possédant un couloir (72) muni d'une sortie, et des moyens servant à faire avancer une file d'agrafes chirurgicales (36, 36a) dans le sens allant vers ladite sortie de manière que les agrafes de tête de la file soient successivement déchargées de la sortie pour être serrées par les mâchoires du dispositif chirurgical, caractérisée en ce qu'un rétracteur (56) est inclus dans le boîtier (50) pour rétracter les agrafes situées en amont de ladite agrafe de tête, dans le sens inverse dudit sens et en les éloignant de ladite sortie avant le serrage de l'agrafe de tête déchargée, afin d'éviter que les agrafes amont ne gênent ledit serrage.

9. Cartouche selon la revendication 8, caractérisée en ce que le rétracteur (56) et les moyens d'avance (60) sont logés dans le boîtier (50), ledit boîtier ayant une section d'extrémité de tête et une section d'extrémité de queue, et un premier et un deuxième couloirs (70, 72) sensiblement parallèles, qui s'étendent tous deux entre lesdites sections d'extrémités, lesdits moyens d'avance comprenant un curseur (60) sollicité par ressort pour solliciter une file d'agrafes contenue dans ledit premier couloir (72) dans la direction longitudinale, vers ladite section d'extrémité de tête, et ledit rétracteur (56) étant logé dans ledit deuxième couloir (70) et possédant une partie de tête qui pénètre dans ledit premier couloir (72) et peut se déplacer dans ce premier couloir entre une position dans laquelle ladite partie de tête est dégagée desdites agrafes et permet à l'agrafe de tête de sortir de ladite section d'extrémité de tête, et une autre position dans laquelle ladite première partie de tête attaque l'agrafe située immédiatement en amont de l'agrafe de tête et la rétracte, par ce moyen, rétracte ladite file d'agrafes, dans le sens allant vers ladite section d'extrémité de queue.

10. Cartouche selon la revendication 9, caractérisée en ce que les moyens d'avance comprennent un curseur (60) qui peut coulisser dans ledit premier couloir (72), et un ressort hélicoïdal (68) à force constante qui attaque ledit curseur (60) pour solliciter ledit curseur dans le sens allant de ladite extrémité de tête et le mettre en contact avec une agrafe de queue de ladite file.

FIG. 6

FIG. 1

*FIG. 2*

*FIG. 4*

*FIG. 3*

*FIG. 5*

0 070 276

FIG. 7

FIG. 10

FIG. 9

FIG. 8

3

FIG. IIA

FIG. IIB

FIG. IIC

4